# EUROPEAN PATENT SPECIFICATION

(11) **EP 4 279 075 B1**
(45) Date of publication and mention of the grant of the patent: **10.07.2024**
(21) Application number: 22461549.2
(22) Date of filing: 12.05.2022
(51) Int. Cl.: A61K 31/513, A61P 15/00, A61K 9/20, A61K 9/28

(54) **A PHARMACEUTICAL COMPOSITION COMPRISING ELAGOLIX**
PHARMAZEUTISCHE ZUSAMMENSETZUNG ENTHALTEND ELAGOLIX
COMPOSITION PHARMACEUTIQUE CONTENANT DE L'ELAGOLIX

(43) Date of publication of application: 22.11.2023
(73) Proprietor: Zaklady Farmaceutyczne Polpharma S.A., 83-200 Starogard Gdanski (PL)
(72) Inventor: Skoczen, Przemyslaw, 83-010 Straszyn (PL); Hrakovsky, Julia, 80-862 Gdansk (PL)

(56) References cited:
- WO-A1-2021/041608
- US-A1- 2019 054 088
- US-B1- 11 273 128

## Description

The present invention relates to a solid pharmaceutical composition comprising elagolix or a pharmaceutically acceptable salt thereof, a surfactant which is polysorbate, and one or more pharmaceutically acceptable excipient(s). Furthermore, the present invention relates to a pharmaceutical unit dosage form containing such pharmaceutical composition. In addition, a process for the preparation of the pharmaceutical composition and of the unit dosage form containing such pharmaceutical composition is described. Moreover, the invention relates to a medical use of the pharmaceutical composition or a unit dosage form for the treatment of endometriosis.

### Background art

Elagolix is a highly potent, selective, orally available and short-duration non-peptide antagonist of the human gonadotropin-releasing hormone receptor (GnRHR). Elagolix is commercially available in the form of sodium salt. Chemical name of elagolix sodium is sodium 4-({(1R)-2-[5-(2-fluoro-3-methoxyphenyl)-3-{[2-fluoro-6-(trifluoromethyl)-phenyl]methyl}-4-methyl-2,6-dioxo-3,6-dihydropyrimidin-1(2H)-yl]-1-phenylethyl}amino)-butanoate. Elagolix sodium has the following structural formula.

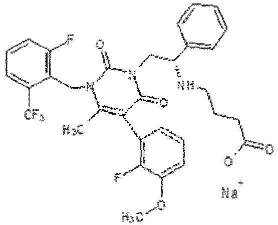

Elagolix has been approved by FDA under the brand name ORILISSA^{®} as the first and only oral gonadotropin-releasing hormone (GnRH) antagonist specifically developed for women with moderate to severe endometriosis pain. The compound is also tested in clinical trials studying the treatment of folliculogenesis, uterine fibroids, heavy uterine bleeding and heavy menstrual bleeding.

ORILISSA^{®} is available as 150 mg and 200 mg film-coated tablets.

WO2009062087 A1 discloses amorphous elagolix sodium and a solid dispersion of amorphous elagolix sodium with a polymer. Manufacturing of solid amorphous mixtures of elagolix sodium with polymers such as polyvinylpyrrolidone and hydroxypropylmethylcellulose is aimed at stabilizing amorphous elagolix sodium.

WO2017221144 A1 relates to a solid dispersion of elagolix sodium. Solid dispersions are said to offer an opportunity to improve the performance profile of a pharmaceutical composition comprising the active substance.

WO2018189213 A1 relates to an amorphous solid dispersion comprising elagolix sodium and at least one silicon-based inorganic compound to prevent deliquescence of elagolix sodium upon contact with moisture to be used in the pharmaceutical composition.

WO2017007895 A1 discloses that the amorphous form of elagolix sodium is hygroscopic and has poor flowability (ffc < 2) and low bulk density (< 0,25 g/mL). Acoustic mixing technology is proposed to obtain granulated amorphous elagolix sodium to improve flowability.

WO2019036712 A1 relates to a pharmaceutical composition comprising elagolix or it's pharmaceutically acceptable salt, an anti-gelling agent in particular sodium carbonate monohydrate and at least one additional excipient.

Sodium carbonate monohydrate as a particular anti-gelling agent is an essential excipient reducing the formation of lactam degradation product of elagolix in the composition.

WO2019036713 A1 relates to a high drug load solid pharmaceutical composition comprising a melt-processed mixture of elagolix or a pharmaceutically acceptable salt thereof, and at least one pharmaceutically acceptable meltable binder. It is again reported there that elagolix sodium has low bulk density and poor flow properties, which present challenges in blending and compression. This problem is overcome by providing a solid pharmaceutical composition comprising elagolix sodium miscible with a binder in a solid matrix. The solid pharmaceutical composition is manufactured by melt-processing such as by melt extrusion or by melt granulation. However, for that process elagolix must be relatively moisture free which is very difficult to achieve and due to the use of high energy from the shear forces and high temperature elagolix might undergo thermal degradation. Moreover, an excipient without proper thermoplastic properties cannot be used and the process requires a highly complex equipment.

WO2020020999 A1 relates to a pharmaceutical composition in the form of a tablet comprising granulated elagolix sodium with a filler, a disintegrant and further excipients.

WO2021180862 A1 discloses a pharmaceutical composition comprising elagolix sodium and pharmaceutically acceptable excipients comprising a super disintegrant and an alkalizer or a non-ionic surfactant such as poloxamer P188.

Further tablet compositions comprising elagolix are disclosed in WO 2021/041608 A1, US 2019/054088 A1 and US 11 273 128 B1.

Amorphous active pharmaceutical ingredients are often used in the development of pharmaceutical solid formulations due to factors such as improved solubility and dissolution rate compared to crystalline forms. However, it has been reported that the amorphous form of elagolix sodium is hygroscopic, and that using it to make pharmaceutical forms has drawbacks such as low content uniformity, low flowability, poor storage stability, sticking during the formulation, and a poor dissolution profile, which could reduce the drug's bioavailability. Therefore, there is a need to provide a pharmaceutical composition comprising elagolix or a pharmaceutically acceptable salt thereof, that is simple to prepare, has high content uniformity, allows for efficient production of unit dosage forms in terms of time and cost, is stable, has an optimal dissolution profile in comparison to the reference product ORILISSA^{®}, and is readily bioavailable.

It has unexpectedly been found that the pharmaceutical composition of the present invention is less complex and that no specific excipient, such as sodium carbonate, is required in the composition, nor is it necessary to manufacture solid dispersions for use in the solid dosage form production process.

The pharmaceutical composition of the present invention is thus simple to prepare, has a high content uniformity, high flowability, allows to avoid or reduce sticking during manufacturing process, is cost and/or time effective, and ensures stability and optimal dissolution profile when compared to the reference product ORILISSA^{®}.

### Summary of the invention

The object of the present invention is to provide a pharmaceutical composition comprising elagolix or a pharmaceutically acceptable salt thereof, a surfactant and one or more pharmaceutically acceptable excipients, wherein the surfactant is polysorbate and wherein polysorbate is present in an amount of from 0,5 % to 3% by weight, based on the total weight of the pharmaceutical composition.

Another object of the present invention is a pharmaceutical unit dosage form containing such pharmaceutical composition.

Another object of the present invention is a process for the preparation of the pharmaceutical composition and of a pharmaceutical unit dosage form containing such pharmaceutical composition.

Yet another object of the present invention is a medical use of said pharmaceutical unit dosage form for the treatment of endometriosis.

### Figures

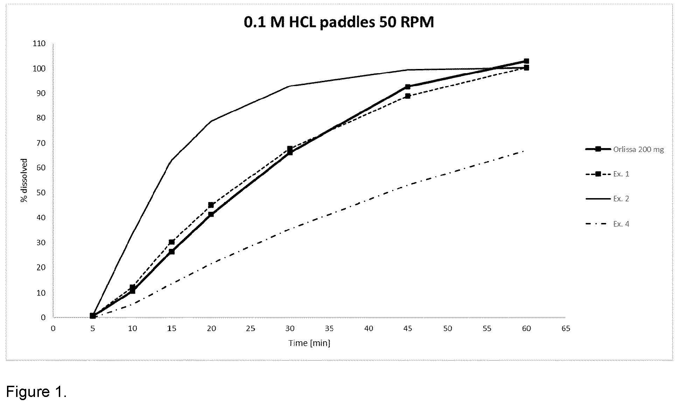

Figure 1 presents dissolution profiles of pharmaceutical unit dosage forms as disclosed in selected Examples versus a reference product Orilissa^{®}.

### Detailed description of the invention

The first object of the present invention is a pharmaceutical composition comprising elagolix or a pharmaceutically acceptable salt thereof, a surfactant and one or more pharmaceutically acceptable excipients, wherein the surfactant is polysorbate and wherein polysorbate is present in an amount of from 0,5 % to 3% by weight, based on the total weight of the pharmaceutical composition.

The pharmaceutical composition of the present invention is suitable for preparing oral unit dosage forms and surprisingly, the oral dosage forms containing such pharmaceutical compositions have good stability and optimal dissolution profile when compared to the reference product ORILISSA^{®}. The composition developed by the present inventors is simple and does not require the use of complex preparation procedures as those disclosed in the prior art, in particular, it does not require the preparation of solid dispersions. Furthermore, the composition does not require the presence of an anti-gelling agent, such as sodium carbonate. Moreover, the presence of excipients, such as a pH modifier, turned out to be unnecessary.

In the pharmaceutical composition of the present invention, the surfactant is present in an amount of from 0,5% to 3%, more preferably is comprised between 0,5% and 2% by weight, in each case based on the total weight of the pharmaceutical composition.

In a preferred embodiment of the pharmaceutical composition of the present invention, polysorbate is selected from polysorbate 20, polysorbate 40, polysorbate 60 and polysorbate 80.

In the most preferred embodiment, the surfactant is polysorbate 80.

In certain embodiment polysorbate 80 is present in a powder form containing 45-65% by weight of polysorbate 80 and 35-55% by weight magnesium aluminometasilicate, based on the total weight of the powder form of polysorbate 80. Such powder is prepared by adsorption of polysorbate 80, which has a form of viscous liquid, on a porous support magnesium aluminometasilicate. Such form is commercially available under the trade name Sepitrap^{™} 80 from Seppic.

In one embodiment of the pharmaceutical composition of the present invention, elagolix or a pharmaceutically acceptable salt thereof is present in an amount which corresponds to from 20 to 50% by weight of elagolix acid, based on the total weight of the pharmaceutical composition. More preferably, elagolix or a pharmaceutically acceptable salt thereof is present in an amount which corresponds to from 25% to 45% by weight of elagolix acid, based on the total weight of the pharmaceutical composition. Even more preferably, elagolix or a pharmaceutically acceptable salt thereof is present in an amount which corresponds to from 30% to 40% by weight of elagolix acid, based on the total weight of the pharmaceutical composition. Most preferably, elagolix in the pharmaceutical composition of the invention is present as elagolix sodium, and in particular as elagolix sodium in amorphous form as disclosed in WO2009062087 A1.

The particular pharmaceutical excipients used for preparing the pharmaceutical composition of the present invention are known in the art and can be chosen by a skilled person depending on their function. Reference can be made in this context to the Handbook of Pharmaceutical Excipients, ed. by R.C. Rowe et al, 6th edition, Pharmaceutical Press (2009).

According to one embodiment of the pharmaceutical composition of the present invention, one or more pharmaceutically acceptable excipient(s) are selected from a group consisting of a filler, a disintegrant, a binder, a pH modifier and a lubricant.

In a preferred embodiment of the pharmaceutical composition of the present invention, the filler is present in an amount of from about 20% to about 76%, more preferably is comprised between 30% and 60% by weight based on the total weight of the pharmaceutical composition.

The filler is a water soluble filler, a water insoluble filler or a mixture thereof.

In the preferred variant of the above embodiment, the filler is water soluble filler and is selected from lactose monohydrate, mannitol, sorbitol and xylitol.

In another preferred variant of the above embodiment, the filler is water insoluble filler and is selected from celluloses such as microcrystalline cellulose or powdered cellulose, and inorganic calcium salts such as calcium phosphate.

In a particular variant of the above embodiment, the filler is selected from mannitol, sorbitol, microcrystalline cellulose and a mixture thereof.

In the most preferred variant of the above embodiment, the filler is mannitol. Mannitol as a filler in combination with polysorbate, in particular with polysorbate 80, resulted in a particular improvement of stability of the composition according to the present invention.

In one embodiment of the pharmaceutical composition of the present invention, the disintegrant is present in an amount of from about 2,5% to about 15%, more preferably is comprised between 5% and 12,5% by weight based on the total weight of the pharmaceutical composition.

In a preferred variant of the above embodiment, the disintegrant is selected from croscarmellose sodium, pregelatinized maize starch, low substituted hydroxypropyl cellulose, methacrylic acid polymer with divinylbenzene potassium salt and maltodextrin.

In the most preferred variant of the above embodiment, the disintegrant is pregelatinized maize starch.

In a further embodiment of the pharmaceutical composition of the present invention, the binder is present in an amount of from about 0,5% to about 6%, more preferably is comprised between 1,5% and 4,5% by weight based on the total weight of the pharmaceutical composition.

In the preferred variant of the above embodiment, the binder is selected from polyvinylpyrrolidone such as N-vinylpyrrolidone or polyvinylpyrrolidone-vinyl acetate copolymer; monosaccharide, such as glucose, fructose, disaccharide as saccharose; polysaccharide; acacia gum; cellulose derivative, such as methylcellulose, hydroxypropyl methylcellulose and hydroxypropyl cellulose; tragacanth; sodium alginate and alginate derivative; and gelatine.

In a preferred variant of the above embodiment, the binder is polyvinylpyrrolidone.

In one further embodiment of the pharmaceutical composition of the present invention, the pH modifying agent is present in an amount of from about 1% to about 25%, more preferably is comprised between 2,5% and 20% by weight based on the total weight of the pharmaceutical composition.

In one variant of the above embodiment, the pH modifying agent is selected from magnesium oxide, calcium carbonate, calcium lactate, calcium dibasic phosphate (anhydrous and dihydrate), magnesium carbonate and sodium bicarbonate.

In a preferred variant of the above embodiment, the pH modifying agent is magnesium oxide.

In one embodiment of the pharmaceutical composition of the present invention, the lubricant is present in an amount of from about 0,5% to about 2,5%, more preferably is comprised between 1% and 2% by weight based on the total weight of the pharmaceutical composition.

In one variant of the above embodiment, the lubricant is selected from magnesium stearate, zinc stearate, calcium stearate, stearic acid, sodium stearyl fumarate and talc.

In a preferred variant of the above embodiment, the lubricant is magnesium stearate.

The pharmaceutical composition according to the present invention comprising elagolix or a pharmaceutically acceptable salt thereof, a surfactant and one or more pharmaceutically acceptable excipient(s), wherein the surfactant is polysorbate and wherein polysorbate is present in an amount of from 0,5 % to 3% by weight, based on the total weight of the pharmaceutical composition is simple to prepare, has a high content uniformity, high flowability, allows to avoid or reduce sticking during the composition's manufacturing process, is cost and/or time effective, and ensures stability and optimal dissolution profile when compared to the reference product ORILISSA^{®}.

The pharmaceutical composition of the present invention can be manufactured by any conventional method known to a skilled person, in particular by wet or dry granulation, or by simple blending of solid substances.

Preferably, the pharmaceutical composition of the present invention is manufactured using wet granulation, where a powder blend of pharmaceutical active ingredient with one or more excipients is subject to agglomeration using a granulation liquid. The agglomeration variants are, for example high shear wet granulation and fluidized bed wet granulation.

Herein is also described a wet granulation process for making the pharmaceutical composition of the present invention, wherein said process comprises the steps of:
- premixing the active ingredient and the main portion of the excipients in a mixer to obtain a pre-mixture;
- granulating the pre-mixture by adding the granulation liquid to obtain granules;
- drying the granules in a fluidized bed dryer or a drying oven to obtain dried granules;
- optionally dry sieving of the dried granules;
- mixing the dried granules with the remaining excipient(s), such as a lubricant, in a mixer to obtain the pharmaceutical composition.

Herein is also described a powder blending process for producing a pharmaceutical composition of the present invention, wherein said process comprises the steps of:
- premixing the active ingredient and the main portion of the excipients in a mixer to obtain a pre-mixture;
- optionally dry screening the pre-mixture through a screen in order to segregate cohesive particles and to improve content uniformity;
- mixing the pre-mixture with remaining excipients to obtain the final powder blend.

The pharmaceutical composition manufactured as above is used to prepare pharmaceutical unit dosage forms.

Another object of the present invention is a pharmaceutical unit dosage form comprising the pharmaceutical composition of the present invention. The pharmaceutical composition of the invention is comprised in such a unit dosage form.

In particular, the unit dosage form is selected from tablets, capsules, sachets, stick-type sachets and ampoules.

Preferred unit dosage forms are tablets and hard capsules, for example, gelatine or hydroxypropylmethylcellulose capsules. In a most preferred embodiment, the unit dosage forms are tablets. The tablets may be uncoated or film-coated.

It is obvious to one of ordinary skill that the amount of the composition of the invention, which is compressed into a tablet or encapsulated in a capsule must be sufficient to ensure adequate dose of the active ingredient per unit dosage.

The pharmaceutical unit dosage form according to the present invention may be prepared by methods well-known to the person skilled in the art.

Preparation of tablets and filling of capsules with the pharmaceutical composition of the invention are known technological operations in the field of pharmaceutical technology and can be performed using standard equipment. For example, tableting of the pharmaceutical composition of the invention can be performed using a rotary tablet press. For example, capsules may be filled using a gravity hopper or various types of feeders, depending on the type of encapsulation machine.

Suitable methods of tabletting include compression of the pharmaceutical composition in the form of the powder blend, i.e. direct compression, or compression of the pharmaceutical composition in the form of granules or a mixture of granules with additional excipients. Tablet cores thus obtained can be further coated with a film-coat, preferably non-functional film-coat.

Film coated tablets can be manufactured using traditional techniques, such as spraying a suitable coating mixture onto the surface of the tablet (tablet core) in a coating machine.

In one embodiment of the pharmaceutical unit dosage form of the present invention, the pharmaceutical unit dosage form is a film-coated tablet comprising a tablet core and a film-coating layer applied onto it. The weight of the coating layer generally amounts from about 2% to about 5% of the weight of the tablet core (i.e., before applying said coating layer). The film-coating layer is not intended for providing any modified release formulation but, rather, the pharmaceutical composition of the invention is an immediate release formulation. The function of the film-coating layer in the tablet, if present, is for example, to improve product appearance, to make swallowing easier or improve the stability of the tablet.

Film-coating, as is well known in the art, involves the deposition, usually by spraying a liquid coating mixture, of a thin film of a polymer-based formulation onto the surface of the tablet core. Beside the film-coating polymer, the film-coating layer may also contain auxiliary excipients, such as anti-tacking agents, plasticizers and/or colorants. An anti-tacking agent is a substance able to reduce or prevent adhesion between particles. Plasticizers can be typically added to increase the flexibility of the coating.

Suitable film-coating polymers include, for example, hydroxypropyl methyl cellulose (HPMC), hydroxypropyl cellulose (HPC), polyvinylpyrrolidone (PVP), polyvinyl alcohol (PVA) or copovidone or mixture of thereof. Solid grades of polyethylene glycol can be also used as a film-coating polymer, or can be used as plasticizer in conjunction with a film-forming polymer. Other suitable plasticizers are for example propylene glycol (macrogol), diethyl phthalate or triethyl citrate.

Common anti-tacking agents that can be added to the coating mixture are, for example, calcium silicate, magnesium silicate, colloidal silicon dioxide, magnesium stearate, calcium stearate, magnesium oxide or talc.

Furthermore, the coating mixture may also comprise pharmaceutically acceptable colorants, for example, iron oxide pigments or titanium dioxide.

Typical film-coat for a tablet according to the present invention comprises:

| Component | Amount (% by weight, based on the total weight of the film-coat) |
|---|---|
| Film-forming polymer | 30 - 70% |
| One or more plasticizers | 1 - 22% |
| One or more anti-tacking agents | 5 - 30% |
| One or more colorants | 0 - 30% |
| Optionally additional additives | ad 100% |

The pharmaceutical unit dosage form according to the present invention has dissolution properties such that after 45 minutes at least 75%, preferably at least 80% by weight of the pharmaceutical active ingredient is dissolved.

The pharmaceutical unit dosage form according to the present invention has a high content uniformity, preferably within a range of from 85 to 115%, more preferably of from 90 to 110%, even more preferably of from 95 to 105% by weight with regards to the pharmaceutical active ingredient. The content uniformity can be determined in a standard test, for example as described in Pharmacopeia, using for example randomly selected 10 pharmaceutical unit dosage forms,.

The procedures employed for preparing the pharmaceutical composition and the dosage forms of the present invention are common, well known procedures for the skilled in pharmaceutical technology, and are disclosed in recognized reference books in the filed, for example, in Aulton's Pharmaceutics. The design and manufacture of medicines, M.E. Aulton and K.M.G. Taylor, editors, Churchill Livingstone Elsevier, Fourth Edition, 2013, or in the book Remington Essentials of Pharmaceutics, L. Felton, editor, Pharmaceutical Press, 2013; or in the book Pharmaceutics Basic principles and application to pharmacy practice. A.K. Dash, S. Singh, and J. Tilman, editors, Academic Press, Elsevier, 2014.

The pharmaceutical unit dosage form obtained from the said process is stable and has an optimal dissolution profile when compared to the reference product ORILISSA^{®}.

Further object of the present invention is a pharmaceutical dosage form comprising a pharmaceutical composition of the present invention for use in the treatment of endometriosis.

### Definitions

Along the present description, as well as in the claims, the singular expressions, generally preceded by the articles "a", "an", or "the", are meant to include also the plural forms, unless the context clearly indicates otherwise. Furthermore, numeric values preceded by the term "about" are meant to include the exact stated value and also a certain variation around such value, namely a variation or ± 5% of the stated amount. Numeric ranges defined by lower and upper endpoints are meant to include also said stated endpoints. The percentages disclosed (%, w%) are always weight percentages, unless clearly stated otherwise.

The term "active ingredient" or "pharmaceutically active ingredient" of a pharmaceutical composition according to the present invention means elagolix or a pharmaceutically acceptable salts thereof, in particular elagolix sodium.

"Polysorbate" has its meaning well-established in the art, i.e. polyoxyethylene sorbitan fatty acid ester (see: Handbook of Pharmaceutical Excipients, ed. by R.C. Rowe et al, 6th edition, Pharmaceutical Press (2009)).

### Experimental part

### Measuring instruments, conditions and protocols:

### Dissolution Profile

Dissolution studies were performed using a USP apparatus 2 (paddle apparatus). The samples were tested in 900 mL of 0,1M HCL solution at 37,0°C +/- 0,5°C and the paddle rotation speed was set at 50 rpm. Dissolution study was conducted for 60 min and the samples were analyzed using the HPLC method.

### Active ingredient

Elagolix sodium used in the examples is in its amorphous form.

The following examples describe the present invention in detail, but are not to be construed to limit the present invention.

### Example 1

| Ingredients | Quantity (mg/tab) |
|---|---|
| Core | |
| Elagolix sodium | 155,2 (which corresponds to 149,75 mg of elagolix acid) |
| Mannitol | 241,3 |
| Polyvinylpyrrolidone | 13,5 |
| Pregelatinized maize starch | 22,5 |
| Polysorbate 80 | 9,0 |
| Magnesium stearate | 8,5 |
| Film-coat | 15,0 |
| Total | 465,0 |

Film-coat comprises polyvinyl alcohol (PVA) (6,3 mg), macrogol (2,7 mg), titanium oxide (3,3 mg), talc (2,5 mg) and iron oxide (0,2 mg).

### Manufacturing process: Wet granulation

Elagolix sodium, povidone, mannitol and pregelatinized maize starch are screened and subsequently pre-mixed in an appropriate high shear mixer.

The pre-mix is moistened with an aqueous solution of polysorbate 80 and granulated using an appropriate high-shear mixer. The granulate is dried in a fluid bed dryer. Subsequently, the granulate is screened through a suitable sieve.

Pre-screened magnesium stearate is added to the granulate and subsequently blending is performed in an appropriate free-fall blender.

The final blend is compressed into tablet cores using 14x8 mm oval punches.

The tablet core is coated by spraying a coating mixture onto the surface of the tablet (tablet core) in a coating machine.

### Example 2

| Ingredients | Quantity (mg/tab) |
|---|---|
| Core | |
| Elagolix sodium | 155,2 (which corresponds to 149,75 mg of elagolix acid) |
| Mannitol | 233,80 |
| Polyvinylpyrrolidone | 13,5 |
| Pregelatinized maize starch | 22,5 |
| Sepitrap^{™} 80 (45-65% w/w of polysorbate 80 and 35-55% w/w of magnesium aluminometasilicate) | 16,5 (which corresponds to approx. 9,07 mg of polysorbate 80) |
| Magnesium stearate | 8,5 |
| Film-coat | 15,0 |
| Total | 465,0 |

Film-coat comprises hydroxypropylmethylcellulose (5,2 mg), triacetin (0,3 mg), macrogol (0,8 mg) titanium dioxide (4,1 mg), iron oxide (0,1 mg) and lactose monohydrate (4,5 mg).

### Manufacturing process: Direct compression

Elagolix sodium, povidone, mannitol, pregelatinized maize starch and Sepitrap^{™} 80 containing 45-65% w/w of polysorbate 80 and 35-55% w/w of magnesium aluminometasilicate are screened and subsequently pre-mixed in an appropriate blender. Then magnesium stearate is added and blended again.

The final blend is compressed into tablet cores using 14x8 mm oval punches.

The tablet core is coated by spraying a coating mixture onto the surface of the tablet (tablet core) in a coating machine.

### Example 3

| Ingredients | Quantity (mg/tab) |
|---|---|
| Core | |
| Elagolix sodium | 155,2 (which corresponds to 149,75 mg of elagolix acid) |
| Mannitol | 199,8 |
| Polyvinylpyrrolidone | 13,5 |
| Pregelatinized maize starch | 22,5 |
| Magnesium oxide | 45,0 |
| Polysorbate 80 | 5,5 |
| Magnesium stearate | 8,5 |
| Film-coat | 15,0 |
| Total | 465,0 |

Film-coat comprises polyvinyl alcohol (PVA) (6,3 mg), macrogol (2,7 mg), titanium oxide (3,3 mg), talc (2,5 mg) and iron oxide (0,2 mg).

### Manufacturing process: Wet granulation

Elagolix sodium, povidone, mannitol, pregelatinized maize starch and magnesium oxide are screened and subsequently pre-mixed in an appropriate high shear mixer.

The pre-mix is moistened with the aqueous solution of polysorbate 80 and granulated using an appropriate high-shear mixer. The granulate is dried in a fluid bed dryer. Subsequently, the granulate is screened through a suitable sieve.

Pre-screened magnesium stearate is added to the granulate and subsequently blending is performed in an appropriate free-fall blender.

The final blend is compressed into tablet cores using 14x8 mm oval punches.

The tablet core is coated by spraying a coating mixture onto the surface of the tablet (tablet core) in a coating machine.

### Comparative example 4

| Ingredients | Quantity (mg/tab) |
|---|---|
| Core | |
| Elagolix sodium | 155,2 (which corresponds to 149,75 mg of elagolix acid) |
| Mannitol | 250,3 |
| Polyvinylpyrrolidone | 13,5 |
| Pregelatinized maize starch | 22,5 |
| Magnesium stearate | 8,5 |
| Film-coat | 15,0 |
| Total | 465,0 |

Film-coat comprises polyvinyl alcohol (PVA) (6,3 mg), macrogol (2,7 mg), titanium oxide (3,3 mg), talc (2,5 mg) and iron oxide (0,2 mg).

### Manufacturing process: Dry granulation

Elagolix sodium, polyvinylpyrrolidone, mannitol and pregelatinized maize starch are screened and subsequently pre-mixed in an appropriate blender.

The pre-mix is compacted using a suitable roller compactor to form a ribbon, which is crushed to obtain granules using a suitable crusher and sieve.

Pre-screened magnesium stearate is added to the granulate and subsequently blending is performed in an appropriate free-fall blender.

The final blend is compressed into tablet cores using 14x8 mm oval punches.

The tablet core is coated by spraying a coating mixture onto the surface of the tablet (tablet core) in a coating machine.

### Example 5 - Dissolution profiles

The dissolution profile of the reference product ORILISSA^{®} was studied vis-a-vis the tablet comprising polysorbate 80 as in Example 1, tablet comprising Sepitrap^{™} 80 as in Example 2, and tablet without polysorbate 80 or sodium carbonate as in the Comparative example 4.

| Time [min] | ORILISSA^{®} 200 mg | Ex.1 | Ex.2 | Comparative Ex.4 |
|---|---|---|---|---|
| | % dissolved | % dissolved | % dissolved | % dissolved |
| 5 | 0,8 | 0,7 | 1,3 | 0,0 |
| 10 | 10,7 | 12,3 | 33,5 | 5,3 |
| 15 | 26,4 | 30,3 | 63,1 | 13,5 |
| 20 | 41,4 | 45,0 | 78,8 | 21,5 |
| 30 | 66,2 | 67,8 | 92,9 | 35,4 |
| 45 | 92,7 | 88,9 | 99,6 | 53,1 |
| 60 | 102,9 | 100,4 | 100,3 | 67,1 |

The dissolution profile results show that the composition containing polysorbate 80 as in Example 1 and Sepitrap^{™} 80 as in Example 2 have optimal dissolution profile when compared to the reference product ORILISSA^{®} when compared with the composition manufactured without addition of sodium carbonate (anti-gelling agent) and without polysorbate 80 (Comparative example 4).

### Example 6 - Stability studies

The stability of the reference product ORILISSA^{®} was studied along with the tablets prepared in Example 1, 2 and Comparative example 4 under accelerated stability conditions by measuring the percentage of impurities at the beginning (T0) and after 2 weeks at 60°C/50% RH and at 60°C/80% RH. All samples were stored in these conditions in an open dish (without primary packaging). Analysis was performed using HPLC. All values relating to content of impurities are expressed in %.

| | ORILISSA^{®} 200 mg tablet | | |
|---|---|---|---|
| Impurities | Initial (T0) | 2 weeks 60°C/50% RH | 2 weeks 60°C/80% RH |
| RRT | | | |
| 0,59 | 0,05 | 0,05 | |
| 0,66 | 0,05 | 0,05 | 0,06 |
| 1,24 | 0,05 | 0,1 | 0,1 |
| 1,64 | 0,12 | 0,16 | 0,15 |
| 1,88 | | 0,05 | 0,1 |
| 2,24 | 0,08 | 0,13 | 0,13 |
| Total impurities | 0,35 | 0,54 | 0,54 |

| | Example 1 | | |
|---|---|---|---|
| Impurities | Initial (T0) | 2 weeks 60°C/50% RH | 2 weeks 60°C/80% RH |
| RRT | | | |
| 0,69 | 0,03 | 0,04 | 0,03 |
| 1,14 | | | |
| 1,24 | 0,03 | 0,03 | 0,03 |
| 1,64 | | | |
| 1,88 | | 0,03 | 0,04 |
| 2,05 | | | |
| 2,06 | | | |
| 2,16 | 0,03 | 0,05 | 0,05 |
| Total impurities | 0,09 | 0,15 | 0,15 |

| | Example 2 | | |
|---|---|---|---|
| Impurities | Initial (T0) | 2 weeks 60°C/50% RH | 2 weeks 60°C/ 80% RH |
| RRT | | | |
| 0,69 | 0,03 | | |
| 1,14 | | 0,08 | 0,07 |
| 1,24 | 0,03 | | |
| 1,64 | | 0,04 | 0,05 |
| 1,88 | | | 0,1 |
| 2,05 | | | |
| 2,06 | | | |
| 2,16 | | 0,05 | 0,05 |
| Total impurities | 0,06 | 0,17 | 0,18 |

| | Comparative Example 4 | | |
|---|---|---|---|
| Impurities | Initial (T0) | 2 weeks 60°C/ 50% RH | 2 weeks 60°C/ 80% RH |
| RRT | | | |
| 0,59 | 0,05 | 0,05 | 0,05 |
| 1,3 | | | 0,07 |
| 1,64 | 0,11 | 0,1 | 0,12 |
| 1,88 | | | 0,1 |
| 1,94 | 0,06 | 0,20 | 0,22 |
| 2,11 | | 0,15 | 0,32 |
| 2,14 | 0,04 | | |
| 2,24 | | 0,04 | 0,05 |
| 2,6 | 0,04 | | |
| Total impurities | 0,3 | 0,54 | 0,93 |

The stability study results show that the lactam impurity (RRT 1,94) is not formed at time T0 in the composition containing polysorbate 80 (Example 1) and Sepitrap^{™} 80 (Example 2) and also during the accelerated stability studies performed for 2 weeks at 60°C/50% RH and at 60°C/80% RH.

In the composition manufactured without addition of sodium carbonate (anti-gelling agent) or polysorbate 80 as in Comparative example 4 the lactam impurity is formed at the level of 0,06%, however after the accelerated stability tests performed for 2 weeks at 60°C/50% RH and at 60°C/80% RH the level of the lactam impurity increases 3 times.

## Claims

1. A solid pharmaceutical composition comprising elagolix or a pharmaceutically acceptable salt thereof, a surfactant and one or more pharmaceutically acceptable excipients, wherein the surfactant is polysorbate and wherein polysorbate is present in an amount of from 0,5% to 3% by weight, based on the total weight of the pharmaceutical composition.

2. The pharmaceutical composition according to claim 1 , wherein the surfactant is present in an amount of from 0,5% to 2% by weight, based on the total weight of the pharmaceutical composition.

3. The pharmaceutical composition according to claim 1 or 2, wherein polysorbate is selected from polysorbate 20, polysorbate 40, polysorbate 60 and polysorbate 80.

4. The pharmaceutical composition according to any one of claims 1 - 3, wherein the surfactant is polysorbate 80.

5. The pharmaceutical composition according to claim 4, wherein polysorbate 80 has a powder form containing 45-65% w/w of polysorbate 80 adsorbed at 35-55% w/w magnesium aluminometasilicate.

6. The pharmaceutical composition according to any one of claims 1 - 5, wherein elagolix or a pharmaceutically acceptable salt thereof is present in an amount which corresponds to from 20 to 50% by weight of elagolix acid, based on the total weight of the pharmaceutical composition.

7. The pharmaceutical composition according to any one of claims 1 - 6, wherein the one or more pharmaceutically acceptable excipient(s) are selected from a group consisting of a filler, a disintegrant, a binder, a pH modifier and a lubricant.

8. The pharmaceutical composition according to any one of claims 1 - 7, wherein the filler is present in an amount of from 20% to 76% by weight, based on the total weight of the pharmaceutical composition.

9. The pharmaceutical composition according to any one of claims 1 - 8, wherein the disintegrant is present in an amount of from 2,5% to 15% by weight, based on the total weight of the pharmaceutical composition.

10. The pharmaceutical composition according to any one of claims 1 - 9, wherein the binder is present in an amount of from 0,5 to 6% by weight, based on the total weight of the pharmaceutical composition.

11. The pharmaceutical composition according to any one of claims 1 - 10, wherein the lubricant is present in an amount of from 0,5 to 2,5% by weight, based on the total weight of the pharmaceutical composition.

12. A pharmaceutical unit dosage form comprising a pharmaceutical composition according to any one of claims 1-11.

13. The pharmaceutical unit dosage form according to claim 12 for use in the treatment of endometriosis.

## Patentansprüche

1. Feste pharmazeutische Zusammensetzung, umfassend Elagolix oder ein pharmazeutisch annehmbares Salz davon, ein Tensid und einen oder mehrere pharmazeutisch annehmbare Trägerstoffe, wobei das Tensid Polysorbat ist und wobei das Polysorbat in einer Menge von 0,5 Gew.-% bis 3 Gew.-% vorliegt, basierend auf dem Gesamtgewicht der pharmazeutischen Zusammensetzung.

2. Pharmazeutische Zusammensetzung nach Anspruch 1, wobei das Tensid in einer Menge von 0,5 Gew.-% bis 2 Gew.-% vorliegt, basierend auf dem Gesamtgewicht der pharmazeutischen Zusammensetzung.

3. Pharmazeutische Zusammensetzung nach Anspruch 1 oder 2, wobei das Polysorbat aus Polysorbat 20, Polysorbat 40, Polysorbat 60 und Polysorbat 80 ausgewählt ist.

4. Pharmazeutische Zusammensetzung nach einem der Ansprüche 1-3, wobei das Tensid Polysorbat 80 ist.

5. Pharmazeutische Zusammensetzung nach Anspruch 4, wobei das Polysorbat 80 eine Pulverform aufweist, die 45-65 Gew.-% Polysorbat 80 enthält, das bei 35-55 Gew.-% adsorbiert ist.

6. Pharmazeutische Zusammensetzung nach einem der Ansprüche 1-5, wobei Elagolix oder ein pharmazeutisch annehmbares Salz davon in einer Menge vorliegt, die von 20 bis 50 Gew.-% Elagolix-Säure entspricht, basierend auf dem Gesamtgewicht der pharmazeutischen Zusammensetzung.

7. Pharmazeutische Zusammensetzung nach einem der Ansprüche 1-6, wobei der eine oder die mehreren pharmazeutisch annehmbare(n) Trägerstoff(e) ausgewählt ist/sind aus einer Gruppe bestehend aus einem Füllstoff, einem Sprengmittel, einem Bindemittel einem pH-Modifizierungsmittel und einem Schmiermittel.

8. Pharmazeutische Zusammensetzung nach einem der Ansprüche 1-7, wobei der Füllstoff in einer Menge von 20 Gew.-% bis 76 Gew.-% vorliegt, basierend auf dem Gesamtgewicht der pharmazeutischen Zusammensetzung.

9. Pharmazeutische Zusammensetzung nach einem der Ansprüche 1-8, wobei das Sprengmittel in einer Menge von 2,5 Gew.-% bis 15 Gew.-% vorliegt, basierend auf dem Gesamtgewicht der pharmazeutischen Zusammensetzung.

10. Pharmazeutische Zusammensetzung nach einem der Ansprüche 1-9, wobei das Bindemittel in einer Menge von 0,5 bis 6 Gew.-% vorliegt, basierend auf dem Gesamtgewicht der pharmazeutischen Zusammensetzung.

11. Pharmazeutische Zusammensetzung nach einem der Ansprüche 1-10, wobei das Schmiermittel in einer Menge von 0,5 bis 2,5 Gew.-% vorliegt, basierend auf dem Gesamtgewicht der pharmazeutischen Zusammensetzung.

12. Pharmazeutische Einheitsdosierungsform, umfassend eine pharmazeutische Zusammensetzung nach einem der Ansprüche 1-11.

13. Pharmazeutische Einheitsdosierungsform nach Anspruch 12 zur Verwendung bei der Behandlung von Endometriose.

## Revendications

1. Composition pharmaceutique solide contenant de l'élagolix ou un sel pharmaceutiquement acceptable de celui-ci, un tensioactif et un ou plusieurs excipients pharmaceutiquement acceptables, dans laquelle le tensioactif est du polysorbate et dans laquelle le polysorbate est présent en une quantité de 0,5 % à 3 % en poids, sur la base du poids total de la composition pharmaceutique.

2. Composition pharmaceutique selon la revendication 1, dans laquelle le tensioactif est présent en une quantité de 0,5 % à 2 % en poids, sur la base du poids total de la composition pharmaceutique.

3. Composition pharmaceutique selon la revendication 1 ou 2, dans laquelle le polysorbate est choisi parmi le polysorbate 20, le polysorbate 40, le polysorbate 60 et le polysorbate 80.

4. Composition pharmaceutique selon l'une quelconque des revendications 1 à 3, dans laquelle le tensioactif est le polysorbate 80.

5. Composition pharmaceutique selon la revendication 4, dans laquelle le polysorbate 80 a une forme de poudre contenant 45 à 65 % p/p de polysorbate 80 adsorbé à hauteur de 35 à 55 % p/p d'aluminométasilicate de magnésium.

6. Composition pharmaceutique selon l'une quelconque des revendications 1 à 5, dans laquelle l'élagolix ou un sel pharmaceutiquement acceptable de celui-ci est présent en une quantité qui correspond à 20 à 50 % en poids d'acide d'élagolix, sur la base du poids total de la composition pharmaceutique.

7. Composition pharmaceutique selon l'une quelconque des revendications 1 à 6, dans laquelle les un ou plusieurs excipients pharmaceutiquement acceptables sont choisis dans un groupe constitué d'une charge, d'un désintégrant, d'un liant, d'un modificateur de pH et d'un lubrifiant.

8. Composition pharmaceutique selon l'une quelconque des revendications 1 à 7, dans laquelle la charge est présente en une quantité de 20 % à 76 % en poids, sur la base du poids total de la composition pharmaceutique.

9. Composition pharmaceutique selon l'une quelconque des revendications 1 à 8, dans laquelle le désintégrant est présent en une quantité de 2,5 % à 15 % en poids, sur la base du poids total de la composition pharmaceutique.

10. Composition pharmaceutique selon l'une quelconque des revendications 1 à 9, dans laquelle le liant est présent en une quantité de 0,5 à 6 % en poids, sur la base du poids total de la composition pharmaceutique.

11. Composition pharmaceutique selon l'une quelconque des revendications 1 à 10, dans laquelle le lubrifiant est présent en une quantité de 0,5 à 2,5 % en poids, sur la base du poids total de la composition pharmaceutique.

12. Forme posologique unitaire pharmaceutique comprenant une composition pharmaceutique selon l'une quelconque des revendications 1 à 11.

13. Forme posologique unitaire pharmaceutique selon la revendication 12, destinée à être utilisée dans le traitement de l'endométriose.
